# FASCICULE DE BREVET EUROPEEN

(11) **EP 1 453 440 B1**
(45) Date de publication et mention de la délivrance du brevet: **30.09.2009**
(21) Numéro de dépôt: 02801127.8
(22) Date de dépôt: 12.12.2002
(51) Int. Cl.: A61F 2/16

(54) **CASSETTE ET INJECTEUR DE LENTILLE INTRAOCULAIRE SOUPLE ET PROCEDE D INJECTION DE TELLES LENTILLES**
KASSETTE UND INJEKTIONSVORRICHTUNG FÜR EINE WEICHE INTRAOKULARLINSE SOWIE VERFAHREN ZUR INJEKTION DERSELBEN
CASSETTE AND INJECTOR FOR FLEXIBLE INTRAOCULAR LENS AND METHOD FOR INJECTING SUCH LENSES

(30) Priorité: 12.12.2001 FR 0116040
(43) Date de publication de la demande: 08.09.2004
(73) Titulaire: Ioltechnologie-Production, 17180 Perigny (FR)
(72) Inventeur: TOURRETTE, Philippe, F-17220 La Jarrie (FR); BERNARD, Pascal, F-17137 Nieul sur Mer (FR); RAQUIN, Vincent, F-17000 La Rochelle (FR)
(74) Mandataire: Kurtz, Laurent Charles Edmond
(86) Numéro de dépôt international: PCT/FR2002/004329
(87) Numéro de publication internationale: WO 2003/049645

(56) Documents cités:
- WO-A-96/03924
- PATENT ABSTRACTS OF JAPAN vol. 2000, no. 21, 3 août 2001 (2001-08-03) & JP 2001 104363 A (CANON STAR KK), 17 avril 2001 (2001-04-17) cité dans la demande
- PATENT ABSTRACTS OF JAPAN vol. 2002, no. 04, 4 août 2002 (2002-08-04) & JP 2001 340374 A (CANON STAR KK), 11 décembre 2001 (2001-12-11) cité dans la demande
- PATENT ABSTRACTS OF JAPAN vol. 2000, no. 21, 3 août 2001 (2001-08-03) & JP 2001 104347 A (CANON STAR KK), 17 avril 2001 (2001-04-17) cité dans la demande

## Description

La présente invention concerne le conditionnement et l'injection de lentilles intraoculaires (LIO) et plus particulièrement des lentilles intraoculaires réalisées en matériau souple.

Une lentille intraoculaire, également appelée implant intraoculaire, comporte une partie optique pour la correction d'amétropies et une partie haptique qui supporte la partie optique dans l'emplacement désiré dans l'oeil qui peut être la chambre antérieure ou la chambre postérieure, pour remplacer le cristallin atteint de la cataracte ou pour apporter une correction avec un cristallin intact.

Les premiers implants intraoculaires avaient la partie optique en matériau rigide et notamment en PMMA qui impliquaient une incision de taille importante correspondant au minimum au diamètre de la partie optique, c'est-à-dire au moins 6 mm. Dans les implants intraoculaires entièrement ou partiellement en matériau souple, la partie haptique peut être pliée ou enroulée, et d'une manière générale, déformée pour permettre son introduction à travers une incision sclérocornéenne de longueur inférieure à la moitié du diamètre de la partie optique.

De nombreux dispositifs sont connus pour le pliage des implants intraoculaires. Leur utilisation est souvent malaisée et ne permet pas de réduire la largeur effective de la partie optique à une valeur inférieure à la moitié du diamètre de la partie haptique.

On connaît également des injecteurs qui permettent d'enrouler ou plier en plusieurs endroits la partie optique, de sorte que la largeur effective de la partie optique est inférieure à la moitié du diamètre de la partie optique. De plus, l'injecteur permet un meilleur contrôle de l'introduction de l'implant, que ce soit dans la chambre antérieure ou la chambre postérieure, et on peut l'injecter d'une seule main.

Les implants ainsi que les injecteurs doivent être stérilisés par des moyens appropriés en fonction des matériaux dans lesquels ils sont réalisés. En effet, certains implants intraoculaires en acrylique hydrophile sont actuellement stérilisés dans une solution aqueuse en autoclave par stérilisation vapeur.

D'autres implants, et notamment ceux réalisés en PMMA, acrylique hydrophobe, bimatériaux de PMMA et acrylique hydrophobe ou en silicone, sont actuellement stérilisés par l'oxyde d'éthylène (ETO). D'autres encore, et notamment des implants réalisés en bimatériaux de PMMA et acrylique hydrophile, doivent être stérilisés par irradiation par rayonnement gamma.

Avant stérilisation à la vapeur en autoclave, l'implant intraoculaire est introduit dans un flacon contenant une solution saline aqueuse qui est ensuite scellé hermétiquement avant l'introduction dans l'autoclave. Le flacon sert de conditionnement pour l'implant.

Il en est de même pour la stérilisation par rayonnement gamma, c'est-à-dire que l'implant intraoculaire est introduit dans un flacon contenant une solution aqueuse et fermé hermétiquement avant de subir l'irradiation.

Enfin, des implants réalisés en acrylique hydrophobe ou bimatériaux de PMMA et acrylique hydrophobe ou en silicone sont stérilisés à sec dans un récipient perméable à l'oxyde d'éthylène et ensuite conditionnés à sec dans un conteneur de forme appropriée.

Les injecteurs sont le plus souvent réalisés soit en polypropylène, soit en polyéthylène basse densité. Les éléments des injecteurs réalisés dans ces matériaux sont stérilisés à l'oxyde d'éthylène sous blister à opercule perméable à l'ETO indépendant du conteneur utilisé pour l'implant intraoculaire lui-même.

Au moment de l'utilisation, on enlève l'injecteur de son conditionnement puis on extrait l'implant intraoculaire du flacon ou autre conteneur pour ensuite le charger dans l'injecteur. Cette opération complique l'utilisation des injecteurs et constitue un risque supplémentaire pour l'intégrité de l'implant, notamment lors du transfert avec des pinces, du conditionnement à une zone de chargement de l'injecteur.

Il a été décrit dans le document WO/96/03924 un dispositif d'insertion de lentilles intraoculaires souples utilisant un compartiment intégré dans le corps de l'injecteur comme dispositif de conditionnement pour le transport et le stockage de la lentille dans un état suspendu et sans contrainte. Selon une forme de réalisation, une première partie du compartiment est formée d'une seule pièce avec le corps tubulaire et une seconde partie est simplement posée sur la première partie. Un tel compartiment ne peut pas en lui-même être utilisé comme un dispositif de conditionnement. Pour ce faire, une canule est rapportée autour du compartiment et attachée au corps du piston de l'injecteur. Dans une autre forme de réalisation, le compartiment est intégré à la canule.

Il en est de même pour les lentilles intraoculaires des demandes de brevet japonais publiées sous les numéros JP-2001104363, JP-2001104347, JP-2001340374 qui sont simplement posées entre deux éléments qui ne peuvent pas constituer un conditionnement pour la stérilisation.

Lorsque l'implant est réalisé en silicone ou en acrylique hydrophobe, l'ensemble constitué par l'implant, le corps de l'injecteur et la canule rapportée sur ce dernier peut être stérilisé à l'ETO. Mais un tel ensemble ne peut être conservé pour une période limitée (environ six mois). En effet, la canule comporte, sous-jacent sur la paroi intérieure un agent de glissement, tel que le stéarate de glycérol, les dérivés de polyamide, les polyéthoxyéthers d'alcool gras, l'ester de polyol, les amines éthoxylées qui sont co-moulés avec la matière constitutive de la canule. Au-delà d'une période limitée, l'agent de glissement initialement imprégné dans le matériau de la canule migre en surface sur la paroi intérieure de la canule dans des quantités telles que l'implant traversant la canule se couvre d'agent de glissement, affectant ainsi sa transparence et entraînant des résidus d'agent dans l'oeil.

Lorsque l'implant est réalisé en acrylique hydrophile ou bimatériaux, l'implant et l'ensemble constitué du corps de l'injecteur et de la canule, ou la canule seule, il ne peut pas être stérilisé avec le conditionnement réalisé typiquement en polypropylène, puisque la canule n'est pas stérilisable à haute température dans un autoclave et un tel implant ne peut pas être stérilisé à l'ETO.

L'objet de la présente invention est de remédier aux problèmes des dispositifs de conditionnement et d'injection d'implants intraoculaires souples, permettant la stérilisation et/ou le stockage de l'implant sur une longue durée, ainsi que le chargement plus facile de l'implant dans un injecteur.

Selon un premier aspect de l'invention, il est prévu une cassette de lentille intraoculaire pour injecteur de lentille, du type comportant un corps tubulaire et un piston mobile dans le corps tubulaire pour faire avancer une lentille vers une canule d'injection, la cassette étant conformée pour être chargée dans le corps tubulaire et comportant un logement pour une lentille intraoculaire, une zone d'entrée pour le piston, une zone de sortie pour l'implant, caractérisée en ce que la cassette constitue un conditionnement de stérilisation de la lentille intraoculaire et présente ses propres moyens de fermeture pour enfermer la lentille.

Une telle cassette constitue également un conditionnement permettant un stockage de l'implant à longue durée. En effet, il peut être réalisé en un matériau, dont la stérilisation est compatible avec celle de la lentille intraoculaire, et ce quel que soit le matériau dans lequel l'implant est réalisé. Le corps de l'injecteur et la canule peuvent être réalisés en un matériau différent de celui de la cassette, stérilisés et conditionnés selon un protocole distinct de celui mis en oeuvre pour la cassette et l'implant.

Selon une forme de réalisation préférée, la cassette comporte un premier élément ou coquille monté mobile sur un second élément ou coquille entre une position d'ouverture et une position de fermeture permettant, le cas échéant, à l'utilisateur d'ouvrir la cassette pour vérifier la lentille intraoculaire, voire pour le prélever en vue de l'implanter sans avoir recours à l'injection. A cette fin, les moyens de fermeture sont de préférence un moyen d'encliquetage déverrouillable.

Selon une variante, les moyens de fermeture assurent la fermeture permanente de la cassette empêchant tout accès à l'implant, sauf au moyen du dispositif d'injection. Une telle cassette peut être munie de fenêtres transparentes pour inspecter l'implant avant chargement dans l'injecteur.

De préférence, quelque soit le mode de réalisation de la lentille, acrylique hydrophile, bi-matériaux hydrophile/ PMMA ou hydrophobe / PMMA ou silicone ou encore acrylique hydrophobe, la cassette est réalisée par exemple en résine polyétherimide et est stérilisable par le mode de stérilisation adaptée au matériau de l'implant.

Pour les lentilles intraoculaires en acrylique hydrophile, la cassette peut être réalisée en un matériau stérilisable en autoclave ou par irradiation par rayonnement gamma, par exemple en résine polyétherimide.

Pour les lentilles intraoculaires réalisées en bi-matériaux dont un matériau hydrophile souple et un matériau rigide, tel que le PMMA, la cassette est réalisée en un matériau stérilisable par irradiation par rayonnement gamma, par exemple en résine polyétherimide.

Lorsque la lentille est réalisée en silicone ou acrylique hydrophobe, la cassette est réalisée en un matériau stérilisable par l'ETO, par exemple en résine polyétherimide.

Selon un autre aspect de l'invention, la cassette et la lentille intraoculaire d'une part, et l'injecteur, d'autre part, peuvent être stérilisés et conditionnés selon un même protocole. La cassette enfermant la lentille intraoculaire peut être même chargée dans l'injecteur et l'ensemble stérilisé selon ce protocole.

Ce protocole peut théoriquement mettre en oeuvre la stérilisation par l'ETO, mais ce n'est pas sans inconvénient. D'une part, la stérilisation par l'ETO implique un dégazage de l'objet pendant une durée relativement longue, de l'ordre de 15 jours pour atteindre un seuil d'oxyde d'éthylène acceptable, et d'autre part, lorsque la canule de l'injecteur est stérilisée par ce procédé, la durée de stockage est très fortement limitée dans le temps, comme déjà indiqué.

Mais selon cet aspect de l'invention, le protocole de stérilisation mettra en oeuvre une stérilisation en milieu humide par rayonnement gamma. En effet, ce type de stérilisation peut être mis en oeuvre pour des lentilles intraoculaires en bimatériaux dont un matériau hydrophile souple et un matériau rigide, tel que le PMMA, ou en simple matériau, et notamment acrylique hydrophile ou PMMA, d'une part, et une cassette réalisée en résine, notamment en polyétherimide, et un injecteur en polyéthylène ou polypropylène, d'autre part. De manière surprenante, il a été constaté que l'injecteur peut être stérilisé par rayonnement gamma avec sa canule d'injection comportant sur la paroi intérieure un agent de glissement sans risque de migration de l'agent de glissement sur la surface de la paroi intérieure de la canule ni risque de contamination de la solution aqueuse et partant, de la lentille intraoculaire. La stérilisation par rayonnement gamma de la cassette avec la lentille intraoculaire et l'ensemble de l'injecteur, effectuée dans un flacon scellé soit avec la cassette chargée dans l'injecteur, soit avec la cassette séparée de celui-ci pour chargement ultérieur.

Selon un autre aspect de l'invention, est prévu un injecteur d'implant intraoculaire adapté à être chargé avec une telle cassette sur la trajectoire d'un piston mobile et aligné avec la canule. De préférence, la cassette est introduite par coulissement transversal dans le corps du piston et est encliquetée dans la position de chargement.

Selon encore un aspect de l'invention, il est prévu un procédé pour l'injection d'implants intraoculaires au moyen d'un dispositif d'injection comprenant un corps tubulaire et un piston mobile comprenant les étapes suivantes : on prépare un implant intraoculaire et une cassette pour le logement de l'implant intraoculaire, la cassette ayant une zone d'entrée de piston et une zone de sortie pour l'implant ; on stérilise l'implant intraoculaire et la cassette après avoir enfermé l'implant dans la cassette ; on stérilise le dispositif d'injection et on introduit la cassette fermée dans le corps tubulaire de l'injecteur avec la zone d'entrée pour le piston alignée avec ce dernier.

De préférence, l'implant est introduit et enfermé dans la cassette, introduit dans un flacon contenant une solution aqueuse saline qui est alors fermé hermétiquement, la stérilisation de la cassette avec l'implant s'effectuant en autoclave ou par rayonnement gamma, pour des implants en acrylique hydrophile ou en bimatériaux souple/rigide d'un acrylique hydrophile souple et d'un matériau rigide, tel que le PMMA.

De préférence, la stérilisation du dispositif d'injection est effectuée concomitamment avec la cassette et la lentille intraoculaire et dans le même flacon. La cassette peut même être chargée dans l'injecteur et ensuite enfermée dans le flacon contenant la solution aqueuse saline. Bien que le chargement de l'implant dans la cassette avant stérilisation soit de loin préféré, selon une variante, l'implant n'est enfermé dans la cassette qu'après stérilisation.

Lorsque l'injecteur est stérilisé non pas par rayonnement gamma mais à l'ETO, la canule d'éjection peut être conditionnée indépendamment du corps tubulaire et du piston afin d'allonger la durée de stockage.

L'invention sera mieux comprise et d'autres avantages de celle-ci apparaîtront à la lumière de la description qui va suivre, donnée à titre d'exemple et faite en référence aux dessins annexés sur lesquels :
- la figure 1 est une vue en perspective d'une cassette de lentille intraoculaire selon une première forme de réalisation de la présente invention en position ouvert avec un implant intraoculaire logé dans un de ses éléments ou coquilles ;
- la figure 2 est une vue en perspective partiellement arrachée de la cassette de la figure 1 en position fermée ;
- la figure 3 est une vue de dessus de la cassette des figures 1 et 2 à plus petite échelle avec un injecteur, avant l'introduction de la cassette et du piston dans le corps tubulaire de l'injecteur ;
- la figure 4 est une vue de côté du corps tubulaire de l'injecteur pris isolément ;
- la figure 5 est une vue de l'injecteur lors de l'éjection de l'implant à travers la canule ;
- la figure 6 est une vue en section suivant la ligne VI-VI de la figure 5 pour illustrer le blocage de la cassette dans le corps tubulaire;
- la figure 7 est une vue en perspective de la cassette, lors du début de l'avancement de l'implant dans la cassette au moyen de la tige du piston ;
- la figure 8 est analogue à la figure 7, au début de l'entrée de l'implant dans le goulet de la canule ; et
- la figure 9 est une vue analogue à celle de la figure 2 pour une variante où la cassette est munie d'un dispositif amovible de blocage de la lentille intraoculaire et d'une patte de préhension.

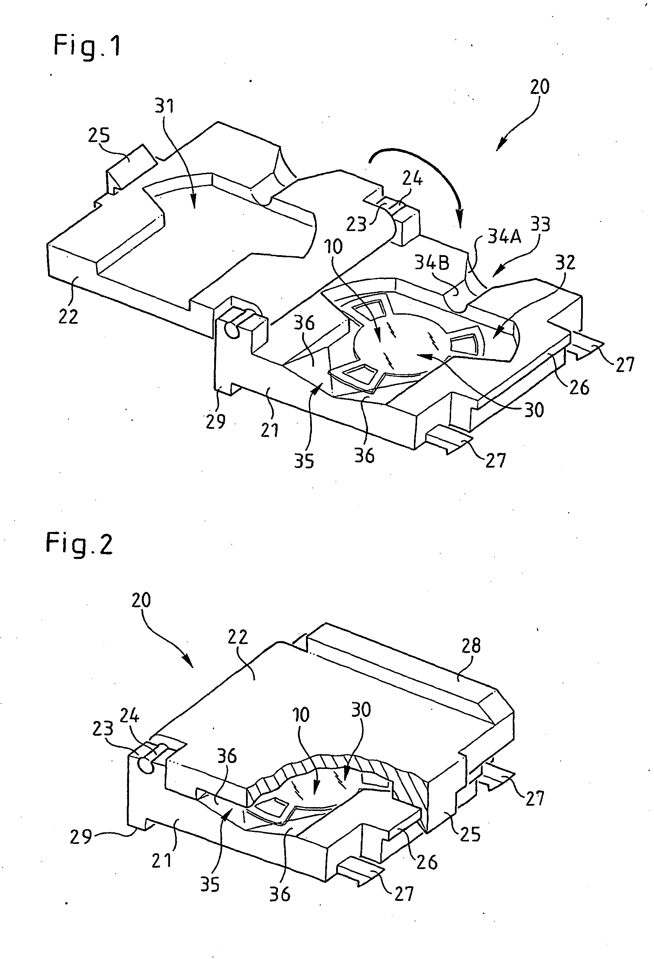

Selon une première forme de réalisation de la présente invention, une cassette 20 est représentée en position ouverte et fermée, respectivement sur les figures 1 et 2. Selon cette forme de réalisation, la cassette comprend un premier élément ou coquille qui constitue une base 21 et un second élément ou coquille qui constitue le couvercle 22. De préférence, chacun de ces éléments ou coquilles est de structure monobloc moulé en matière plastique, de préférence au moins partiellement transparente. Chacun des éléments ou coquilles 21 et 22 a un contour général rectangulaire et une épaisseur réduite par rapport aux autres dimensions. De part et d'autre du côté arrière de la base est disposé un bloc comportant un siège 23 élastiquement déformable susceptible de recevoir un tourillon 24 de forme complémentaire, et notamment cylindrique, disposé de part et d'autre du côté arrière du couvercle latéralement en saillie dans des échancrures. L'assemblage se fait en passant des tourillons à travers la fente d'accès des sièges 23, l'élasticité du siège assurant le maintien du tourillon pour le pivotement du couvercle.

Sur le bord avant du couvercle 22 est disposé un crochet 25 qui coopère avec un rebord 26 s'étendant sur le côté avant de la base 21 dès la fermeture de la cassette. Le crochet 25 fléchit vers l'avant lorsqu'il rencontre le rebord 26 et ensuite s'encliquète sous le rebord pour verrouiller la cassette. Dans cette forme de réalisation, les moyens de fermeture de la cassette sont des moyens d'encliquetage.

Selon une variante non représentée, les moyens de fermeture assurent la fermeture permanente et inviolable de la cassette après l'introduction de l'implant, de sorte que celui-ci ne peut être manipulé qu'au moyen de l'injecteur correspondant. Avec une telle forme de réalisation, la base et/ou le couvercle sont réalisés partiellement ou entièrement en une matière transparente afin de permettre l'inspection de l'implant à travers la (ou les) paroi(s) transparente(s).

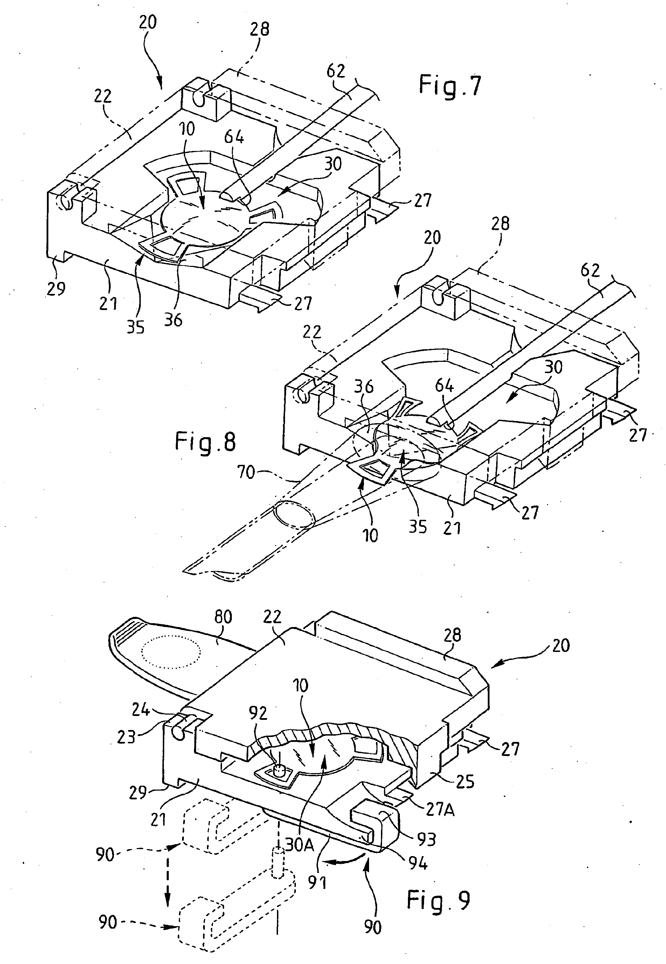

La base 21 et, le cas échéant, le couvercle 22, comportent sur la face intérieure une cavité 30, 31, de contour et de profondeur pour loger une lentille intraoculaire 10. Le logement peut être conçu pour loger toute configuration de lentilles intraoculaires, telle que par exemple celle illustrée dans la forme de réalisation de la figure 9 ou, en revanche, conçu pour loger une configuration de lentille intraoculaire déterminée, et ce quelle que soit la correction de la partie optique..Pour une forme de lentille intraoculaire telle qu'illustrée, le logement a une configuration sensiblement complémentaire du contour de l'implant. Le fond 32 de la cavité est de préférence plan, tel qu'illustré.

Dans la forme de réalisation illustrée à la figure 1, la cavité 30 de la base 21 définissant le logement en position fermée de la cassette, est destinée à recevoir une lentille intraoculaire 10 à trois haptiques plates fenêtrées ou non et tel que décrit dans le brevet français N° 2.745.711. A cette fin, la cavité 30 a un contour général en T avec le bord avant des bras latéraux légèrement oblique pour retenir deux des trois haptiques plates pour empêcher ainsi tout mouvement substantiel de l'implant. La partie de la cavité qui constitue la jambe du T a une largeur légèrement supérieure au diamètre de l'optique de la lentille intraoculaire. La largeur de la lentille intraoculaire au niveau des deux pattes arrière disposées dans la barre de la cavité en T est, dans cette forme de réalisation, supérieure à la largeur de la jambe du T de la cavité. Dans une telle configuration, les parois obliques de la barre de la cavité en T à la jonction avec les parois de la tige de la cavité retiennent la lentille intraoculaire par ses haptiques pour empêcher la sortie inopportune de la lentille de la cassette.

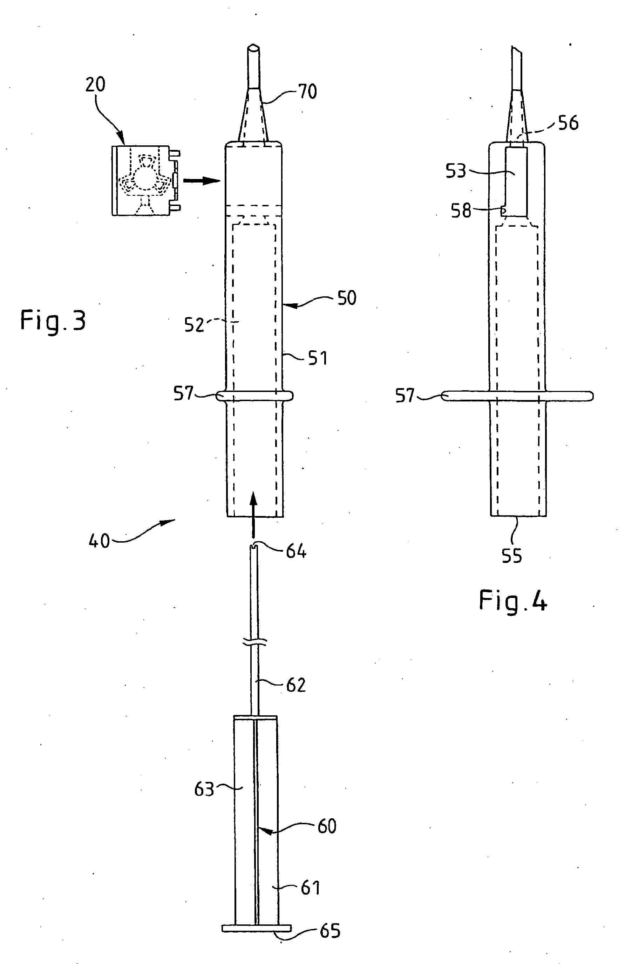

Dans cette forme de réalisation, la troisième haptique plate se trouve du côté sortie 35 de la cavité, en communication avec une canule ou embout 70 (voir figures 3 à 5). La cavité 30 comporte deux pans obliques 36 de forme triangulaire, tel qu'illustré, qui constituent une rampe destinée à relever l'implant et déterminent le sens d'enroulement qui a lieu dans la canule, tel que décrit ci-dessus.

Dans le côté opposé à la sortie 35 se trouve l'entrée 33 pour l'extrémité distale de la tige 62 du piston 60 (figure 3). Cette entrée comporte une première zone effilée 34A suivie d'une seconde zone cylindrique 34B. L'ensemble des première et seconde zones de la base et du couvercle à l'entrée de la cassette sert de guidage pour l'extrémité distale de la tige dans la cassette.

Le couvercle 22 comporte une cavité 31 de même contour que la cavité 30 de la base ainsi qu'une première zone effilée et une seconde zone semi-cylindrique identiques à celles de l'entrée 33. La cavité 31 dans le couvercle ne comporte pas des pans obliques 36. Dans une forme de réalisation non illustrée, la cavité 31 peut avoir un contour différent de celui de la cavité 30, voire être supprimée. Dans ce cas, la hauteur de la cavité 30 doit être suffisamment importante compte tenu de la voûte de l'implant à l'état de repos.

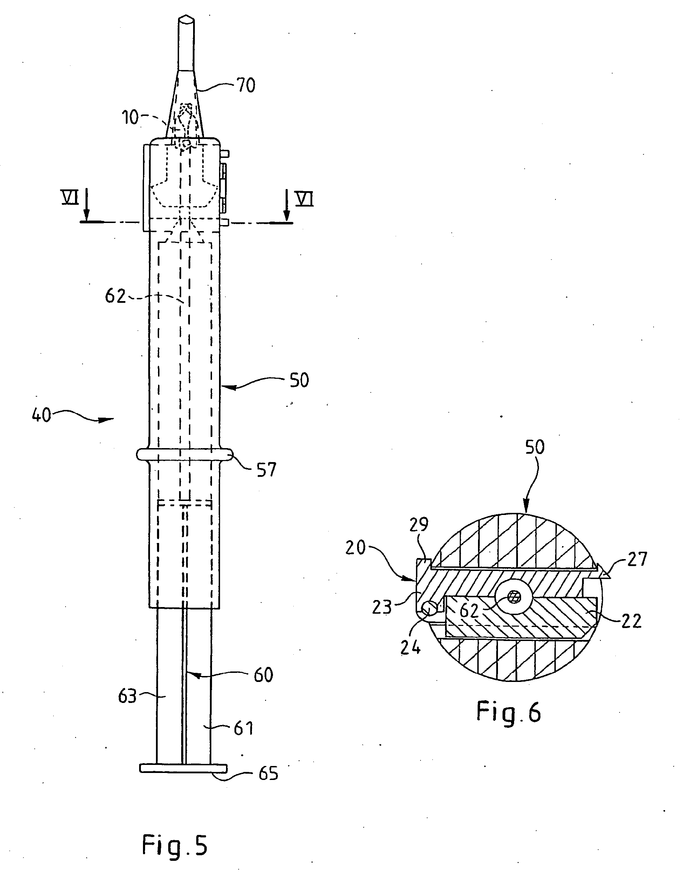

Sur l'extérieur de la base 21, côté arrière, et alignée avec les tourillons, est disposée une nervure 29 qui constitue une butée pour arrêter le coulissement de la cassette dans le corps tubulaire 40 (voir figure 6). Une paire de crochets 27 est située dans des décrochements situés de part et d'autre du rebord 26 et sont adaptés à coopérer avec la surface extérieure du corps tubulaire à la sortie du logement dans celui-ci, de sorte que, lorsque la cassette est introduite dans lé corps tubulaire, les crochets 27 sont déformés et ensuite retrouvent leur position de repos où ils coopèrent avec le bord du corps tubulaire 50 (voir figure 6). Ces crochets 27 constituent des moyens d'encliquetage pour fixer la cassette dans le logement 53 du corps tubulaire. De même, sur l'extérieur du couvercle 22, côté entrée, est disposée une deuxième nervure 28 avec une extrémité avant oblique qui coopère avec une rainure complémentaire 58 communiquant avec le logement 53 dans le corps tubulaire pour guider la cassette lors de l'introduction dans le logement 53 du corps tubulaire, d'une part et pour constituer un moyen détrompeur pour empêcher la mauvaise orientation de la cassette dans le logement du corps tubulaire, d'autre part.

En vue de sa stérilisation et ensuite de son stockage, l'implant est introduit dans la cavité 30 et la cassette fermée par les moyens d'encliquetage constitués par les crochets 25 et le rebord 26. A l'état de repos, et dans la position illustrée à la figure 2, le centre de l'optique est en contact avec le fond 32 de la cavité 30. S'agissant d'un implant ayant une angulation vers l'intérieur de l'ordre de 10°, les haptiques inclinées à cet angle se redressent légèrement vers "le fond" de la cavité .31 du couvercle. De préférence, la hauteur totale de la cavité conjointe 30, 31 sera légèrement supérieure de la hauteur de l'implant passant par son axe optique, de sorte qu'il y a un faible jeu. De même, il y a également un jeu angulaire permettant un léger débattement des éléments haptiques de l'implant par rapport au bord contour de la cavité.

Lorsque l'implant est réalisé en matériau acrylique hydrophile, par exemple un copolymère hydroxyéthylméthacrylate et méthylméthacrylate, il peut être stérilisé à la vapeur en autoclave, soit par rayonnement gamma. La cassette doit être réalisée dans un matériau susceptible d'être stérilisé à la vapeur soit en autoclave, soit par rayonnement gamma, selon le cas. De préférence, la cassette sera réalisée en polyétherimide, commercialisé sous la marque et référence ULTEM 1010F par la société General Electric Company, qui permet la stérilisation par l'un ou par l'autre procédé.

Une variante de la cassette est représentée sur la figure 9. Les mêmes éléments de cette variante sont identifiés par les mêmes références que celles aux figures 1, 2, 6 à 8.

Selon cette forme de réalisation, la base 21 comporte sur son côté arrière une patte de préhension 80 s'étendant à l'arrière et ayant un bord libre relevé. Grâce à cette patte, la manipulation de la cassette est facilitée et notamment pour son introduction dans le dispositif d'injection. En effet, elle assure une bonne prise de la cassette jusqu'à ce que celle-ci arrive dans la position d'emboîtement dans le dispositif d'injection, tel qu'illustré à la figure 6.

Selon cette forme de réalisation, la cavité 30A n'a pas une configuration en T, de sorte que celle-ci puisse être adaptée à recevoir une lentille intraoculaire de toutes configurations, et notamment avec d'autres formes d'éléments haptiques plats, en J, en C, ou autre. De même, la cavité 30A est à fond plat jusqu'à la sortie, c'est-à-dire qu'elle ne comporte pas de pans obliques d'amorçage d'enroulement. La largeur de la cavité 30A côté sortie est suffisamment importante pour permettre le libre passage de la lentille.

La cassette 20 est munie d'un dispositif amovible de blocage 90 de la lentille 10. Ce dispositif comprend un bras 91 comportant à une extrémité distale une butée 92 apte à coopérer avec la périphérie de l'optique et/ou un des éléments haptiques de la lentille intraoculaire pour empêcher la sortie inopportune de la lentille de la cassette. Le bras 91 comporte à son extrémité proximale un crochet 93 de forme sensiblement complémentaire à une languette 94 sur la base 21 au côté sortie de la cavité 30A. Dans la position de blocage, le crochet 93 est en prise avec la languette 94. Dans cette position, la butée 92 peut coopérer avec l'élément haptique à proximité de la sortie et/ou le bord périphérique de l'optique. Tel qu'illustré pour l'implant à trois haptiques plates fenêtrées, cette butée pénètre à cette fin dans la fenêtre de l'élément optique. La butée 92 passe par un trou s'étendant perpendiculairement au plan général du fond de la cavité 30A pratiqué dans la base et constitue un axe permettant de faire pivoter le bras 91 de la position de blocage dans le sens de la flèche vers une position où le crochet 93 affranchit la languette vers la position illustrée en pointillés où la butée 92 peut être retirée du trou pour libérer la lentille intraoculaire (également illustrée en pointillés).

On remarquera enfin qu'un des crochets 27A est décalé sous le rebord 26 et donc adjacent à la languette 94. La fonction d'une paire de crochets 27, 27A intervient exactement de la même manière que la paire de crochets 27 de la première forme de réalisation.

Il va de soi que la cassette des figures 1, 2 et 6 à 8, avec une cavité en T peut être munie d'un tel dispositif de blocage pour renforcer le maintien de la lentille intraoculaire dans ladite cavité lors de la stérilisation et du stockage.

Avant stérilisation, la cassette renfermant l'implant est introduite dans un flacon (non représenté) fabriqué en polypropylène PPT rempli d'une solution saline appropriée et ensuite refermée hermétiquement par un opercule. Grâce aux ouvertures côté entrée et côté sortie, la solution aqueuse peut circuler à l'intérieur de la cassette et autour de l'implant lors du cycle de stérilisation. La présence de la solution aqueuse à l'intérieur de la cavité 30, 31 évite toute détérioration de l'implant et plus particulièrement de la partie haptique lors des légers débattements de l'implant à l'intérieur de la cavité, tant dans le sens de l'axe optique de l'implant, que dans le sens circonférentiel.

La stérilisation en autoclave est effectuée à environ 120°C, et de préférence à 121°C, pendant environ 30 minutes et de manière générale, conformément à une technique connue en elle-même. Une fois stérilisés, la cassette et l'implant peuvent être conservés pour une durée d'environ deux à cinq ans.

La stérilisation par rayonnement gamma dans une solution saline est effectuée à une dose d'environ 25 kGy permettant la destruction des microorganismes, selon une technique connue en elle-même. Il s'agit d'une dose de préférence conforme à la Pharmacopée française et européenne. D'autres dosages, par exemple de 35 kGy, conformément à la Pharmacopée danoise, peuvent également être utilisés. Après stérilisation, la cassette et l'implant peuvent être conservés également pour une durée de deux à cinq ans. Selon un protocole particulièrement avantageux, la stérilisation de l'injecteur est effectuée en même temps que celle de la cassette et de la lentille. A cette fin, la cassette avec sa lentille et l'injecteur sont introduits dans un flacon étanche rempli de solution saline et ensuite fermés par un opercule thermoscellé.

Les implants réalisés en bimatériaux souple et rigide, notamment en polyméthylméthacrylate (PMMA) et acrylique hydrophile, tel que décrit dans la demande de brevet PCT WO99/65422. Le matériau souple est obtenu à partir de copolymères statistiques méthacrylate de méthyle-méthacrylate d'hydroxyéthylméthacrylate (MMA-HEMA) réticulés par ajout d'un agent multifonctionnel tel que le diméthacrylate de diéthylèneglycol. Le matériau rigide de la lentille est de préférence à base de copolymères PMMA-HEMA réticulés par du diméthacrylate de diéthylèneglycol. Un tel implant bimatériaux doit être stérilisé par rayonnement gamma pour empêcher une opacification ou dénaturation chimique du PMMA.

Enfin, pour les implants réalisés en acrylique hydrophobe ou silicone, la cassette sera réalisée de préférence en ULTEM 1010F commercialisé par la société General Electric, en polypropylène, commercialisé sous les marque et référence Hostalen PPT 1070 Si par la société Hoechst ou en polyéthylène basse densité, commercialisé sous les marque et référence Riblene PE. L'implant enfermé dans sa cassette est conditionné dans un conteneur, tel qu'un blister, à opercule perméable à l'oxyde d'éthylène, et stérilisé à sec dans une chambre à l'oxyde d'éthylène, selon une technique connue en elle-même. Cette stérilisation est suivie d'une phase d'aération pour la désorption et l'évacuation des gaz d'une quinzaine de jours. De tels implants en cassette peuvent être conservés de deux à cinq ans selon le cas.

Le cas échéant, d'autres procédés de stérilisation peuvent être utilisés, tels que stérilisation plasma basse température avec du peroxyde d'hydrogène. Sous l'effet d'une décharge électromagnétique, il y a formation de radicaux libres qui détruisent les liaisons organiques des microorganismes. Dans ce cas, la cassette pourrait être réalisée en polyétherimide Ultem 1010F, polypropylène Hostatel PPT 1070, ou polyéthylène basse densité Riblene PE.

Au moment de l'utilisation, on sort la cassette du flacon ou autre récipient, éventuellement avec le dispositif d'injection lorsqu'ils sont stérilisés ensemble dans le même flacon, on l'introduit directement dans le logement du dispositif d'injection, tel qu'illustré sur les figures 3 à 6.

Le dispositif d'injection ou injecteur 40 est du type seringue et comporte un piston 60, un corps tubulaire 50 et une canule 70 d'éjection d'implant. Le piston et le corps tubulaire seront des composants distincts. La canule est montée de préférence de manière amovible à la sortie 56 du corps tubulaire. La canule peut, en variante, être solidarisée avec le corps tubulaire à la sortie 56. Le piston 60 comporte un élément de guidage cruciforme 61 ayant sensiblement la même largeur que le diamètre intérieur de l'alésage 52 du corps tubulaire 50. L'élément de guidage est prolongé par une tige 62 de petit diamètre de l'ordre de 0,75 à 1,5 mm, et préférentiellement 1,0 mm, suffisamment fine pour limiter le risque de coincement de l'implant entre la tige et la cassette et entre la tige et la canule ou embout. La tige 62 comporte à son extrémité une gorge diamétrale 64 apte à coopérer avec la périphérie de la partie optique de l'implant ou éventuellement une des haptiques de celui-ci, et ce pour l'avancer jusqu'à la sortie de la canule 70. La tête 65 du piston limite l'avancement de la tige par rapport à la sortie de la canule.

L'alésage 52 du corps tubulaire 50 comporte à son extrémité avant une surface tronconique ayant le même angle de cône que la première zone à l'entrée de la cassette et en continuité avec elles et sert également à guider l'extrémité distale de la tige du piston 60. Au-delà de cette surface tronconique et en communication avec celle-ci est un passage transversal traversant de part et d'autre le corps tubulaire 50 et qui constitue un logement 53 pour la cassette 20 et donc a une section longitudinale complémentaire de la section correspondante de la cassette. Ce logement comporte une rainure de guidage 58 complémentaire de la nervure de guidage 29 le long du côté avant du couvercle 22 de la cassette 20. Lorsque la cassette 20 est insérée dans le logement 53, celle-ci est retenue d'une part par les crochets 27 et d'autre part, par la butée 28. La cassette et/ou logement peuvent être munis de moyens empêchant l'extraction de celle-ci et évitant ainsi toute réutilisation de la cassette ou de l'injecteur. A l'extérieur du corps tubulaire 50 est disposée une collerette 57 à environ un tiers de la distance à partir de l'extrémité proximale du corps 50.

Après chargement de la cassette 20 enfermant la lentille intraoculaire 10 dans le logement 53 du corps tubulaire 50, le piston peut alors être introduit et avancé dans l'alésage 52, à travers la zone tronconique à son extrémité avant et la zone tronconique à l'entrée de la cassette et ensuite entrer en contact par sa gorge 64 de largeur plus grande que l'épaisseur correspondante de la zone d'engagement de la partie optique ou le cas échéant, de celle d'un des éléments haptiques afin de guider et d'entraîner l'implant à travers les pans obliques 36 de la cassette constituent une rampe qui servent à relever progressivement l'implant d'un plan parallèle au fond de la cavité 30 dans la base vers le plan général passant par l'axe longitudinal de la canule. En relevant ainsi l'implant, on assure un sens d'enroulement préférentiel, ici avec les éléments haptiques latéraux passant au-dessus de la partie haptique. L'enroulement proprement dit ne commence qu'à l'entrée de la canule, tel qu'illustré à la figure 8 où l'on voit la partie optique s'incurver légèrement, amorçant ainsi l'enroulement. En revanche, dans la variante représentée à la figure 9, où la cavité est à fond plat, il n'y a pas d'amorçage d'enroulement à l'intérieur de la cavité.

Le processus d'enroulement progresse avec l'avancement de l'implant dans la première partie de la canule 70 ayant la forme d'un goulet et se rétrécissant vers une seconde partie cylindrique de la canule à l'extrémité distale de laquelle se trouve la sortie de la canule et donc celle de l'ensemble du dispositif d'injection. Lors de cet avancement de la lentille dans le goulet, l'implant s'enroule sur lui-même en se resserrant progressivement autour d'une ligne diamétrale de l'optique jusqu'à l'arrivée de la portion de partie cylindrique à la sortie de la canule 70. L'angle circonférentiel de l'enroulement en fin de course est supérieur à 360° et, en pratique d'environ 540°. La sortie est légèrement oblique pour faciliter l'éjection de l'implant. La gorge 64 de la tige 62 entraîne alors l'implant jusqu'à la sortie de la canule où celui-ci peut se dérouler dans le segment de l'oeil pour l'implantation de l'implant, c'est-à-dire selon le cas, la chambre antérieure ou le sulcus ciliaire ou le sac capsulaire de la chambre postérieure.

Les composants du dispositif d'injection 40 sont de préférence réalisés en polypropylène, ou polyéthylène basse densité, et peuvent être stérilisés également à l'oxyde d'éthylène sous blister à opercule perméable à ce gaz, dans les conditions habituelles. Dans ce cas, les trois composants de l'injecteur, c'est-à-dire le piston, le corps tubulaire et la canule, peuvent être conditionnés sous un seul blister. Pour améliorer les possibilités de conservation du corps tubulaire et du piston, jusqu'à deux à cinq ans, la canule sera alors un élément distinct du corps tubulaire et conditionnée dans un blister indépendant, et donc avec une période de péremption plus courte (de six mois environ. )

Il va de soi que la présente invention n'est pas limitée aux formes de réalisation décrites ni aux matériaux préférés, mais englobe au contraire toutes variantes de structures et configurations et de matériaux compatibles avec les objets de la présente invention.

## Revendications

1. Cassette de lentille intraoculaire pour injecteur de lentille, du type comportant un corps tubulaire et un piston mobile dans le corps tubulaire pour faire avancer une lentille vers une canule d'injection, la cassette (20) étant conformée pour être chargée dans le corps tubulaire (50) et comportant un logement pour une lentille intraoculaire (10), une zone d'entrée pour le piston (60), une zone de sortie pour la lentille, **caractérisée en ce que** la cassette (20) constitue un conditionnement de stérilisation de la lentille intraoculaire et présente ses propres moyens de fermeture (25 26) pour enfermer la lentille.

2. Cassette selon la revendication 1, **caractérisée en ce que** la cassette comporte un premier élément monté mobile sur un second élément entre une position d'ouverture et une position de fermeture.

3. Cassette selon la revendication 1 ou 2, **caractérisée en ce que** les moyens de fermeture consistent en un moyen d'encliquetage déverrouillable (25, 26).

4. Cassette selon la revendication 1, **caractérisée en ce que** les moyens de fermeture sont un moyen de fermeture permanente de la cassette.

5. Cassette selon l'une quelconque des revendications 1 à 4, pour lentille intraoculaire en acrylique hydrophile, **caractérisée en ce que** la cassette est réalisée en un matériau stérilisable en autoclave.

6. Cassette selon la revendication 5, **caractérisée en ce qu'**elle est réalisée en résine polyétherimide.

7. Cassette selon l'une quelconque des revendications 1 à 4, pour une lentille réalisée en bi-matériaux dont un matériau hydrophile souple et un matériau rigide, **caractérisée en ce que** la cassette est réalisée en un matériau stérilisable par irradiation par rayonnement gamma.

8. Cassette selon l'une quelconque des revendications 1 à 4, pour une lentille réalisée en acrylique hydrophile souple, **caractérisée en ce que** la cassette est réalisée en un matériau stérilisable par irradiation par rayonnement gamma.

9. Cassette selon l'une quelconque des revendications 1 à 4, pour une lentille réalisée en silicone ou acrylique hydrophobe ou en bi-matériaux dont un acrylique hydrophobe souple et un matériau rigide ou acrylique hydrophobe, **caractérisée en ce que** la cassette est réalisée en un matériau stérilisable par oxyde d'éthylène.

10. Cassette selon l'une quelconque des revendications 1 à 9, **caractérisée en ce que** la cassette comporte un moyen d'accrochage (25) au corps tubulaire (50).

11. Cassette selon l'une quelconque des revendications 1 à 10, **caractérisée en ce qu'**elle comprend une butée (92) pour limiter son introduction dans le corps tubulaire.

12. Cassette selon l'une quelconque des revendications 1 à 6 ou 10, 11, en combinaison avec un flacon pour autoclave, **caractérisée en ce que** la cassette enfermant la lentille intraoculaire réalisée en acrylique hydrophile est conditionnée dans le flacon contenant une solution aqueuse et fermé hermétiquement.

13. Cassette selon l'une quelconque des revendications 1 à 5, 7, 8, 10 et 11, en combinaison avec un flacon pour rayonnement gamma, et **caractérisée en ce que** la cassette enfermant la lentille intraoculaire est conditionnée dans un flacon contenant une solution aqueuse et fermé hermétiquement.

14. Cassette selon l'une quelconque des revendications 1 à 5, 9 à 11, en combinaison avec un conteneur à fermeture hermétique, **caractérisée en ce que** la cassette enfermant la lentille intraoculaire réalisée en silicone ou acrylique hydrophobe est conditionnée à sec dans le conteneur.

15. Cassette selon l'une quelconque des revendications précédentes, **caractérisée en ce qu'**elle est munie d'un dispositif de blocage (90) de la lentille dans le logement.

16. Cassette selon la revendication 15, **caractérisée en ce que** le dispositif de blocage (90) de la lentille comprend un bras (91) ayant un crochet (93) qui coopère avec un élément de la cassette dans une position de blocage et une butée (92) qui, dans la position de blocage, pénètre dans le logement et est apte à coopérer avec un élément haptique et/ou la périphérie de la partie optique de la lentille intraoculaire.

17. Injecteur de lentille intraoculaire comportant une cassette selon l'une quelconque des revendications 1 à 16, **caractérisé en ce qu'**il comporte un corps tubulaire (50) et un piston mobile (60) dans le corps tubulaire entre une position d'attente et une position d'injection, et **en ce qu'**il comprend une canule (70) disposée à une extrémité du corps tubulaire à l'opposé du piston, et **en ce que** le corps tubulaire comporte un logement (53) pour recevoir la cassette, de sorte que le logement de la cassette pour la lentille est aligné avec la trajectoire du piston et la canule d'injection.

18. Injecteur selon la revendication 17, **caractérisé en ce que** le logement pour la cassette comporte une glissière (58) s'étendant transversalement par rapport à l'axe général du corps du piston (60).

19. Injecteur selon les revendications 10 et 17, **caractérisé en ce que** la glissière a un seul côté d'entrée de cassette, et **en ce que** le moyen d'accrochage (27) coopère avec le corps tubulaire (50) du côté opposé à l'entrée, lorsque la cassette est en place dans le logement du corps tubulaire.

20. Injecteur selon les revendications 11 et 17 ou 18, **caractérisé en ce que** la butée (28) coopère avec le corps tubulaire (50) du côté d'entrée de la cassette (20).

21. Injecteur selon l'une quelconque des revendications 17 à 20, **caractérisé en ce que** le corps tubulaire (50) et le piston (60) sont réalisés en un matériau stérilisable par l'oxyde d'éthylène et conditionnés ensemble.

22. Injecteur selon la revendication 21, **caractérisé en ce que** la canule (70) comporte un agent de glissement sur son passage interne et conditionné dans un conditionnement distinct de celui du corps tubulaire et du piston (60) et de celui de la cassette enfermant la lentille intraoculaire.

23. Injecteur selon l'une quelconque des revendications 17 à 21, **caractérisé en ce que** la canule (70) est solidaire du corps tubulaire (50).

24. Procédé de conditionnement et de mise en place d'une lentille intraoculaire dans un injecteur selon l'une quelconque des revendications 17 à 23, **caractérisé en ce qu'**il comprend les étapes suivantes :
- on prépare une lentille intraoculaire et une cassette pour le logement de la lentille intraoculaire, la cassette ayant une zone d'entrée de piston et une zone de sortie pour la lentille ;
- on stérilise la lentille intraoculaire et la cassette après fermeture de la lentille dans la cassette,
- on stérilise le dispositif d'injection ; et
- on introduit la cassette fermée dans le corps tubulaire de l'injecteur avec la zone d'entrée pour le piston alignée avec ce dernier.

25. Procédé selon la revendication 24, **caractérisé en ce que** la lentille chargée dans la cassette, d'une part, et l'injecteur d'autre part sont stérilisés selon un même protocole.

26. Procédé selon la revendication 24, **caractérisé en ce que** la cassette renfermant la lentille intraoculaire est chargée dans l'injecteur et l'ensemble qui en résulte est ensuite stérilisé par un même protocole.

27. Procédé selon la revendication 24, **caractérisé en ce que** la lentille intraoculaire est introduite et enfermée dans la cassette; qui est et ensuite introduite dans un flacon contenant une solution aqueuse laquelle étant- alors fermée hermétiquement, et **en ce que** la stérilisation de la cassette avec la lentille s'effectue en autoclave.

28. Procédé selon la revendication 24, **caractérisé en ce que** la lentille intraoculaire est introduite et enfermée dans la cassette, la cassette avec la lentille étant ensuite introduits dans un flacon contenant une solution aqueuse qui est alors fermé hermétiquement, et **en ce que** la stérilisation de la cassette avec la lentille est effectuée par rayonnement gamma.

29. Procédé selon la revendication 28, **caractérisé en ce que** l'injecteur est également introduit dans le flacon contenant la solution aqueuse pour stérilisation concomitante de la cassette et de l'injecteur.

30. Procédé selon la revendication 29, **caractérisé en ce que** la cassette est chargée dans l'injecteur avant leur introduction dans le flacon contenant la solution aqueuse.

31. Procédé selon la revendication 27 ou 28, **caractérisé en ce que** le dispositif d'injection comportant une canule d'injection, le dispositif d'injection étant stérilisé et conditionné indépendamment de la cassette et de la lentille.

32. Procédé selon la revendication 31, **caractérisé en ce que** la canule d'injection est conditionnée séparément de la cassette et de la lentille, d'une part et du reste de l'injecteur, d'autre part.

33. Procédé selon l'une quelconque des revendications 26 à 32, **caractérisé en ce que** l'introduction de la cassette s'effectue par glissement dans une glissière transversale dans le corps tubulaire et **en ce que** la cassette est alors encliquetée en place.

## Claims

1. Intraocular lens cassette for lens injector, of the type comprising a tubular body and a piston mobile in the tubular body to advance a lens toward an injection canula, the cassette (20) being conformed to be loaded into the tubular body (50) and comprising a housing for an intraocular lens (10), an entry region for the piston (60), and an exit region for the lens, **characterised in that** the cassette (20) constitutes packaging for sterilising the intraocular lens and has its own closure means (25, 26) for enclosing the lens.

2. Cassette according to claim 1, **characterised in that** it includes a first member mounted on a second member and mobile between an open position and a closed position.

3. Cassette according to claim 1 or 2, **characterised in that** the closure means comprises unlockable clipping means (25, 26).

4. Cassette according to claim 1, **characterised in that** the closure means comprises means for permanent closure of the cassette.

5. Cassette according to any of claims 1 to 4, for hydrophilic acrylic intraocular lens, **characterised in that** the cassette is made from a material that can be sterilised in an autoclave.

6. Cassette according to claim 5, **characterised in that** it is made from polyetherimide resin.

7. Cassette according to any of claims 1 to 4, for a bimaterial lens made from a hydrophilic flexible material and a rigid material, **characterised in that** the cassette is made from a material that can be sterilised by irradiation with gamma radiation.

8. Cassette according to any of claims to 4, for a lens made of hydrophilic flexible acrylic, **characterised in that** the cassette is made from a material that can be sterilised by irradiation with gamma radiation.

9. Cassette according to any of claims 1 to 4, for a lens made of silicone or hydrophobic acrylic or a bimaterial lens made of a hydrophobic flexible acrylic and a rigid material or a hydrophobic acrylic, **characterised in that** the cassette is made from a material that can be sterilised by ethylene oxide.

10. Cassette according to any of claims 1 to 9, **characterised in that** it includes means (25) for attachment to the tubular body (50).

11. Cassette according to any of claims 1 to 10, **characterised in that** it comprises an abutment (92) for limiting its insertion into the tubular body.

12. Cassette according to any of claims 1 to 6 or 10, 11, in combination with an autoclave flask, **characterised in that** the cassette containing an intraocular lens made of hydrophilic acrylic is packaged in the flask, which contains an aqueous solution and is hermetically sealed.

13. Cassette according to any of claims 1 to 5, 7, 8, 10 and 11, in combination with a flask for gamma irradiation, **characterised in that** the cassette containing the intraocular lens is packaged in the flask, which contains an aqueous solution and is hermetically sealed.

14. Cassette according to any of claims 1 to 5, 9 to 11, in combination with a hermetically sealed container, **characterised in that** the cassette containing the intraocular lens made of silicone or hydrophobic acrylic is packaged dry in the container.

15. Cassette according to any preceding claim, **characterised in that** it has a device (90) for immobilising the lens in the housing.

16. Cassette according to claim 15, **characterised in that** the device (90) for immobilising the lens comprises an arm (91) having a hook (93) that cooperates with an element of the cassette in an immobilising position and an abutment (92) which, in the immobilising position, enters the housing and is adapted to cooperate with a haptic member and/or the periphery of the optical portion of the intraocular lens.

17. Intraocular lens injector comprising a cassette according to any of claims 1 to 16, **characterised in that** it comprises a tubular body (50) and a piston (60) mobile in the tubular body between a waiting position and an injection position, **in that** it comprises a canula (70) disposed at an end of the tubular body opposite the piston, and **in that** the tubular body comprises a housing (53) to receive the cassette so that the housing of the cassette for the lens is aligned with the pathway of the piston and the injection canula.

18. Injector according to claim 17, **characterised in that** the housing for the cassette comprises a slideway (58) extending transversely to the general axis of the body of the piston (60).

19. Injector according to claims 10 and 17, **characterised in that** the slideway has a single cassette entry side, and **in that** the attachment means (27) cooperates with the tubular body (50) on the side opposite the entry side when the cassette is in place in the housing of the tubular body.

20. Injector according to claims 11 and 17 or 18, **characterised in that** the abutment (28) cooperates with the tubular body (50) on the entry side of the cassette (20) .

21. Injector according to any of claims 17 to 20, **characterised in that** the tubular body (50) and the piston (60) are made from a material that can be sterilised by ethylene oxide and are packaged together.

22. Injector according to claim 21, **characterised in that** the canula (70) comprises a lubricant on its internal passage and is packaged in packaging separate from that of the tubular body and the piston (60) and from that of the cassette containing the intraocular lens.

23. Injector according to any of claims 17 to 21, **characterised in that** the canula (70) is fastened to the tubular body (50).

24. Method of packaging and placing an intraocular lens in an injector according to any of claims 17 to 23, **characterised in that** it comprises the following steps:
- an intraocular lens and a cassette for housing the intraocular lens are prepared, the cassette having a piston entry region and an lens exit region;
- the intraocular lens and the cassette are sterilised after enclosing the lens in the cassette;
- the injection device is sterilised; and
- the closed cassette is inserted into the tubular body of the injector with the piston entry region aligned therewith.

25. Method according to claim 24, **characterised in that** the lens loaded into the cassette, on the one hand, and the injector on the other hand are sterilised according to the same protocol.

26. Method according to claim 24, **characterised in that** the cassette containing the intraocular lens is loaded into the injector and the resulting combination is then sterilised according to the same protocol.

27. Method according to claim 24, **characterised in that** the intraocular lens is introduced into and enclosed in the cassette, which is then introduced into a flask containing an aqueous solution that is then hermetically sealed, and **in that** the cassette is sterilised with the lens in an autoclave.

28. Method according to claim 24, **characterised in that** the intraocular lens is inserted into and enclosed in the cassette, the cassette with the lens then being introduced into a flask containing an aqueous solution that is then hermetically sealed, and **in that** the cassette is sterilised with the lens by gamma radiation.

29. Method according to claim 28, **characterised in that** the injector is also introduced into the flask containing the aqueous solution for concomitant sterilisation of the cassette and the injector.

30. Method according to claim 29, **characterised in that** the cassette is loaded into the injector before they are introduced into the flask containing the aqueous solution.

31. Method according to claim 27 or 28, **characterised in that**, the injection device including an injection canula, the injection device is sterilised and packaged independently of the cassette and the lens.

32. Method according to claim 31, **characterised in that** the injection canula is packaged separately from the cassette and the lens, on the one hand, and the remainder of the injector, on the other hand.

33. Method according to any of claims 26 to 30, **characterised in that** the cassette is inserted by sliding it in a transverse slideway in the tubular body and **in that** the cassette is then clipped into place.

## Patentansprüche

1. Kassette für Intraokularlinse für Linseninjektor des Typs, der einen rohrförmigen Körper und in dem rohrförmigen Körper einen beweglichen Kolben enthält, um eine Linse zu einer Injektionskanüle vorzutreiben, wobei die Kassette (20) so beschaffen ist, dass sie in den rohrförmigen Körper (50) geladen werden kann, und einen Aufnahmesitz für eine Intraokularlinse (10), eine Eintrittszone für den Kolben (60) und eine Austrittszone für die Linse umfasst, **dadurch gekennzeichnet, dass** die Kassette (20) eine sterile Verpackung für die Intraokularlinse bildet und ihre eigenen Verschlussmittel (25, 26) aufweist, um die Linse einzuschließen.

2. Kassette nach Anspruch 1, **dadurch gekennzeichnet, dass** die Kassette ein erstes Element umfasst, das an einem zweiten Element zwischen einer geöffneten Stellung und einer geschlossenen Stellung beweglich angebracht ist.

3. Kassette nach Anspruch 1 oder 2, **dadurch gekennzeichnet, dass** die Verschlussmittel aus einem ausrückbaren, entriegelbaren Einrastmittel (25, 26) bestehen.

4. Kassette nach Anspruch 1, **dadurch gekennzeichnet, dass** die Verschlussmittel ein Mittel zum dauerhaften Verschließen der Kassette sind.

5. Kassette nach einem der Ansprüche 1 bis 4 für Intraokularlinse aus hydrophilem Acryl, **dadurch gekennzeichnet, dass** die Kassette aus einem im Autoklaven sterllisierbaren Material hergestellt ist.

6. Kassette nach Anspruch 5, **dadurch gekennzeichnet, dass** sie aus einem Polyetherimid-Harz hergestellt ist.

7. Kassette nach einem der Ansprüche 1 bis 4 für eine Linse, die aus einem Bimaterial hergestellt ist, das ein elastisches hydrophiles Material und ein starres Material umfasst, **dadurch gekennzeichnet, dass** die Kassette aus einem durch Bestrahlung mit Gammastrahlung sterilisierbaren Material hergestellt ist.

8. Kassette nach einem der Ansprüche 1 bis 4 für eine Linse, die aus elastischem hydrophilem Acryl hergestellt ist, **dadurch gekennzeichnet, dass** die Kassette aus einem durch Bestrahlung mit Gammastrahlung sterilisierbaren Material hergestellt ist.

9. Kassette nach einem der Ansprüche 1 bis 4 für eine Linse, die aus Silikon oder aus hydrophobem Acryl oder aus einem Bimaterial, das ein elastisches hydrophobes Acryl und ein starres Material oder ein hydrophobes Acryl umfasst, hergestellt ist, **dadurch gekennzeichnet, dass** die Kassette aus einem durch Ethylenoxid sterilisierbaren Material hergestellt ist.

10. Kassette nach einem der Ansprüche 1 bis 9, **dadurch gekennzeichnet, dass** die Kassette ein Mittel (25) zum Einhaken an dem rohrförmigen Körper (50) umfasst.

11. Kassette nach einem der Ansprüche 1 bis 10, **dadurch gekennzeichnet, dass** sie einen Anschlag (92) umfasst, um ihre Einführung in den rohrförmigen Körper zu begrenzen.

12. Kassette nach einem der Ansprüche 1 bis 6 oder 10, 11 in Kombination mit einem Autoklaven-Fläschchen, **dadurch gekennzeichnet, dass** die Kassette, die die aus hydrophilem Acryl hergestellte Intraokularlinse umschließt, in dem Fläschchen, das eine wässrige Lösung enthält und hermetisch dicht verschlossen Ist, verpackt ist.

13. Kassette nach einem der Ansprüche 1 bis 5, 7, 8, 10 und 11 in Kombination mit einem Fläschchen für Gammastrahlung, **dadurch gekennzeichnet, dass** die Kassette, die die Intraokularlinse umschließt, in einem Fläschchen verpackt ist, das eine wässrige Lösung enthält und hermetisch dicht verschlossen ist.

14. Kassette nach einem der Ansprüche 1 bis 5, 9 bis 11, in Kombination mit einem Behälter mit hermetisch dichtem Verschluss, **dadurch gekennzeichnet, dass** die Kassette, die die aus Silikon oder hydrophobem Acryl hergestellte Intraokularlinse umschließt, in dem Behälter trocken verpackt Ist.

15. Kassette nach einem der vorhergehenden Ansprüche, **dadurch gekennzeichnet, dass** sie mit einer Vorrichtung (90) zum Blockieren der Linse in ihrem Aufnahmesitz versehen ist.

16. Kassette nach Anspruch 15, **dadurch gekennzeichnet, dass** die Linsenblockiervorrichtung (90) einen Arm (91) umfasst, der einen Haken (93), der in der Blockierposition mit einem Element der Kassette zusammenwirkt, und einen Anschlag (92), der in der Blockierposition in den Aufnahmesitz eindringt und dazu ausgelegt ist, mit einem haptischen Element und/oder mit dem Umfang des optischen Teils der Intraokularlinse zusammenzuwirken, aufweist.

17. Injektor für Intraokularlinse, der eine Kassette nach einem der Ansprüche 1 bis 16 umfasst, **dadurch gekennzeichnet, dass** er einen rohrförmigen Körper (50) und einen Kolben (60), der in dem rohrförmigen Körper zwischen einer Ruheposition und einer Injektionsposition beweglich ist, umfasst und dass er eine Kanüle (70) umfasst, die an einem Ende des rohrförmigen Körpers gegenüber dem Kolben angeordnet ist, und dass der rohrförmige Körper einen Aufnahmesitz (53) zum Aufnehmen der Kassette umfasst, derart, dass der Aufnahmesitz der Kassette für die Linse auf Bahn die des Kolbens und der Injektionskanüle ausgerichtet ist.

18. Injektor nach Anspruch 17, **dadurch gekennzeichnet, dass** der Aufnahmesitz für die Kassette eine Gleitschiene (58) umfasst, die in Bezug auf die allgemeine Achse des Kolbenkörpers (60) quer verläuft.

19. Injektor nach den Ansprüchen 10 und 17, **dadurch gekennzeichnet, dass** die Gleitschiene eine einzige Kassetteneintrittsseite besitzt und dass das Einhakmittel (57) mit dem rohrförmigen Körper (50) auf der dem Eingang gegenüberliegenden Seite zusammenwirkt, wenn die Kassette in dem Aufnahmesitz des rohrförmigen Körpers angeordnet ist.

20. Injektor nach den Ansprüchen 11 und 17 oder 18, **dadurch gekennzeichnet, dass** der Anschlag (28) mit dem rohrförmigen Körper (50) auf der Eintrittsseite der Kassette (20) zusammenwirkt.

21. Injektor nach einem der Ansprüche 17 bis 20, **dadurch gekennzeichnet, dass** der rohrförmige Körper (50) und der Kolben (60) aus einem durch Ethylenoxid sterilisierbaren Material hergestellt sind und gemeinsam verpackt sind.

22. Injektor nach Anspruch 21, **dadurch gekennzeichnet, dass** die Kanüle (70) auf ihrem inneren Durchlass ein Gleitmittel aufweist und in einer Verpackung verpackt ist, die von jener des rohrförmigen Körpers und des Kolbens (60) und von jener der die Intraokularlinse umschließenden Kassette verschieden ist.

23. Injektor nach einem der Ansprüche 17 bis 21, **dadurch gekennzeichnet, dass** die Kanüle (70) mit dem rohrförmigen Körper (50) fest verbunden ist.

24. Verfahren zum Verpacken und Anordnen einer Intraokularlinse in einem Injektor nach einem der Ansprüche 17 bis 23, **dadurch gekennzeichnet, dass** es die folgenden Schritte umfasst:
- Vorbereiten einer Intraokularlinse und einer Kassette für die Aufnahme der Intraokularlinse, wobei die Kassette eine Kolbeneintrittszone und eine Austrittszone für der Linse besitzt;
- Sterilisieren der Intraokularlinse und der Kassette nach dem Einschließen der Linse in der Kassette,
- Sterilisieren der Injektionsvorrichtung; und
- Einführen der geschlossenen Kassette in den rohrförmigen Körper des Injektors, wobei die Eingangszone für den Kolben auf diesen letzteren ausgerichtet ist.

25. Verfahren nach Anspruch 24, **dadurch gekennzeichnet, dass** die in die Kassette geladene Linse einerseits und der Injektor andererseits gemäß demselben Protokoll sterilisiert werden.

26. Verfahren nach Anspruch 24, **dadurch gekennzeichnet, dass** die Kassette, die die Intraokularlinse einschließt, in den Injektor geladen wird und die daraus sich ergebende Gesamtheit anschließend gemäß demselben Protokoll sterilisiert wird.

27. Verfahren nach Anspruch 24, **dadurch gekennzeichnet, dass** die Intraokularlinse in die Kassette eingesetzt und darin eingeschlossen wird, wobei die Kassette anschließend in ein Fläschchen eingesetzt wird, das eine wässrige Lösung enthält und dann hermetisch dicht verschlossen wird, und dass die Sterilisation der Kassette mit der Linse im Autoklaven erfolgt.

28. Verfahren nach Anspruch 24, **dadurch gekennzeichnet, dass** die Intraokularlinse in die Kassette eingesetzt und darin eingeschlossen wird, wobei die Kassette mit Linse anschließend in ein Fläschchen eingesetzt wird, das eine wässrige Lösung enthält und das dann hermetisch dicht verschlossen wird, und dass die Sterilisation der Kassette mit der Linse durch Gammastrahlung erfolgt.

29. Verfahren nach Anspruch 28, **dadurch gekennzeichnet, dass** der Injektor ebenfalls in das die wässrige Lösung enthaltende Fläschchen eingesetzt wird, um die Kassette und den Injektor auf gleiche Weise zu sterilisieren.

30. Verfahren nach Anspruch 29, **dadurch gekennzeichnet, dass** die Kassette in den Injektor geladen wird, bevor sie in das die wässrige Lösung enthaltende Fläschchen eingesetzt wird.

31. Verfahren nach Anspruch 27 oder 28, **dadurch gekennzeichnet, dass** die Injektionsvorrichtung dann, wenn sie eine Injektionskanüle enthält, unabhängig von der Kassette und von der Linse sterilisiert und verpackt wird.

32. Verfahren nach Anspruch 31, **dadurch gekennzeichnet, dass** die Injektionskanüle getrennt von der Kassette und der Linse einerseits und vom Rest des Injektors andererseits verpackt wird.

33. Verfahren nach einem der Ansprüche 26 bis 30, **dadurch gekennzeichnet, dass** das Einsetzen der Kassette durch Gleiten in einer transversalen Gleitschiene in den rohrförmigen Körper erfolgt und dass die Kassette dann an ihrem Ort eingerastet wird.
